Europäisches Patentamt

(19) European Patent Office

Office européen des brevets

(11) Publication number: **0 062 620**
**B1**

(12) # EUROPEAN PATENT SPECIFICATION

(45) Date of publication of patent specification: **18.09.85**

(51) Int. Cl.⁴: **G 01 N 21/53**, G 01 N 33/34

(21) Application number: **82850046.2**

(22) Date of filing: **10.03.82**

(54) **Method of measuring the content of fibrillary particles in a pulp.**

(30) Priority: **19.03.81 SE 8101741**

(43) Date of publication of application:
**13.10.82 Bulletin 82/41**

(45) Publication of the grant of the patent:
**18.09.85 Bulletin 85/38**

(84) Designated Contracting States:
**DE FR GB SE**

(56) References cited:
**DE-C-2 923 946**
**US-A-4 078 863**
**US-A-4 159 639**

**ABSTRACTS BULLETIN OF THE INDUSTRY OF**
**PAPER CHEMISTRY, vol. 43, no. 9, March 1973,**
**page 929, no. 9560, K. PRZYBYSZ et al.: "Fines**
**in pulp"**
**ABSTRACTS BULLETIN OF THE INDUSTRY OF**
**PAPER CHEMISTRY, vol. 48, no. 10, April 1978,**
**page 1087, no. 10144; J. HILL et al.: "S.T.F.I.**
**long-fiber content meter in process control**
**applications"**

(73) Proprietor: **SVENSKA**
**TRÄFORSKNINGSINSTITUTET**
**Drottning Kristinas väg 37 F, 53-69**
**S-114 28 Stockholm (SE)**

(72) Inventor: **Karlsson, Håkan Ingvar**
**Skrakvägen 85**
**S-184 00 Åkersberga (SE)**
Inventor: **Pettersson, Jan Gustav Thorulf**
**Marknadsvägen 147**
**S-183 34 Täby (SE)**

(74) Representative: **Illum, Leif-Otto**
**Svenska Cellulosa Aktiebolaget SCA**
**Kungsgatan 33**
**S-111 56 Stockholm (SE)**

(56) References cited:
**ABSTRACTS BULLETIN OF THE INDUSTRY OF**
**PAPER CHEMISTRY, vol. 49, no. 6, December**
**1978, page 550, no. 4991; S. TRAIDUK et al.:**
**"Method of determining degree of fibrillation of**
**pulp"**
**APPLIED OPTICS, vol. 18, no. 11, 1st June 1979,**
**pages 1763-1769, New York (USA); K.**
**CASHDOLLAR et al.: "Three-wavelength light**
**transmission technique to measure smoke**
**particle size and concentration"**

Courier Press, Leamington Spa, England.

## Description

This invention relates to a method of measuring the content of fibrillary particles in a pulp containing also fibres of larger dimensions.

For obtaining by beating (refining) a pulp with certain desired properties, it is necessary to control the beating process. For this control usually so-called freeness testers are used which determine the freeness of drainage property of the pulp. For automatic control of the beating, however, these testers are utilized to a small extent, because the freeness does not constitute an unambigious measure of the quality and operability of the pulp. The dewatering, and thereby the freeness measure, by their nature depend on various factors, such as fines and fibre treatment.

The present invention implies that the surface of the proportion of the content of fibrillary particles, which content here is called crill, in the beaten material is determined. From this the relative crill content, i.e. the crill proportion of the pulp, can be determined. By combining the crill proportion with a measure of drainage properties of the pulp, preferably measured by a highspeed dewatering method, it was found possible to calculate the mechanical properties of the fibres by means of a mathematic model. When paper qualities and operability properties of the pulp were related to the crill proportion and said calculated mechanical properties of the fibres, distinct interrelations could be observed. From these interrelations it was possible to read optimum operation conditions. These interrelations further can be utilized for automatically controlling the beating process.

At the beating of cellulose material, the fibres are affected in different ways. Fibrillation, for example, occurs, i.e. the fibrils of the fibre walls are more or less exposed. These fibrillary particles are called crill particles. Entirely separated fibrils are called free crill and exposed but not entirely separated fibrils are called bound crill. The crill particles have a diameter which is about one hundred times smaller than the fibre diameter.

The crill particles have a very large surface in relation to their weight and, therefore, affect to a high degree the dewatering properties of the pulp as well as the porosity and mechanical properties of the paper product. The dewatering properties are especially affected by the free crill content.

From US Patent Specification 4 078 863 a method is known for measuring the content of fibrillary particles in a pulp in accordance with the prior art portion of claim 1. This known method is based on the fact that if a flowing liquid with suspended material is illuminated, the light incident to the liquid will be scattered by its reflection from the particles present in the liquid. The intensity of light scattered in a definite direction becomes greater as the size of the particle increases from which the light has been scattered. By detecting the scattered light and converting it into an electric signal which is analysed the concentration of particles above and below a certain size can be determined. However, by this method it is not possible to distinguish fibrillary particles (crill) from fine fraction in general.

The present invention implies a measurement, at which the capacity of the fibrillary particles (crill) to absorb and diverge light rays (absorbance) at different wavelengths is utilized for determining the content of fibrillary particles in the pulp. According to the invention the illuminating light has a first wavelength selected so as to correspond to the mean diameter of the fibrillary particles and a second wavelength selected so as to be longer than the first wavelength but shorter than the mean diameter of the fibres. From the detected intensities of the light transmitted through the pulp at the first and second wavelengths a first and second absorbance value are derived and the content of the fibrillary particles is determined from the difference between the absorbance values at the first and second wavelengths.

The invention is described in greater detail in the following, with reference to some examples and to the drawings, in which

Fig. 1 shows the principle of the measurement according to the invention by using two light sources,

Fig. 2 shows an alternative, at which only one light source is used, and

Fig. 3 shows how the absorbance at a constant particle concentration varies with the particle diameter at two particular wavelengths of light.

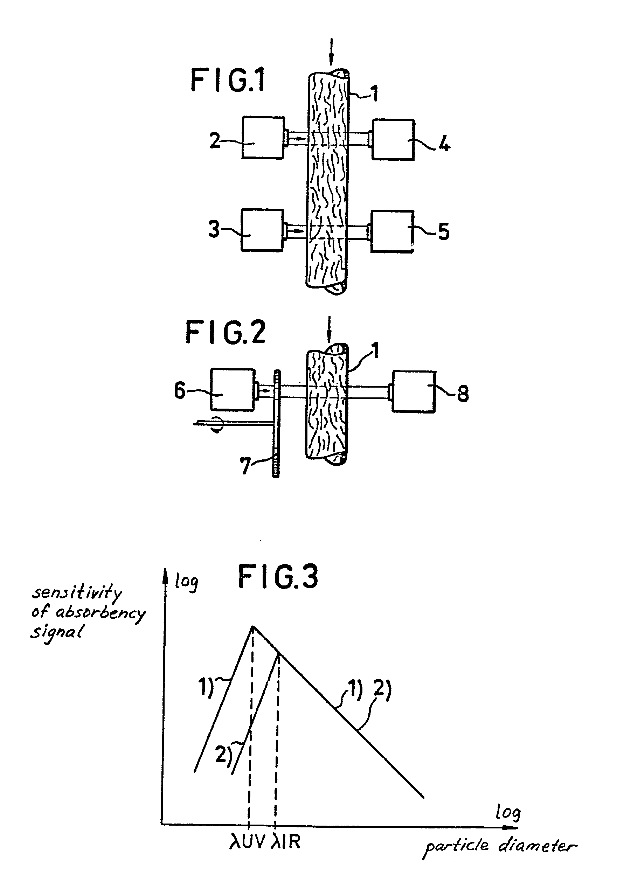

A sample of the pulp to be measured is passed through a passageway 1 preferably in the form of a glass cell. According to Fig. 1, the pulp is transilluminated by light of different wavelengths from two light sources 2 and, respectively, 3. The transmitted light is collected by two light detectors 4 and, respectively 5. According to Fig. 2, a light source 6 and a rotary filter 7 are used which alternatingly permit the passage of light of two different wavelengths. The transmitted light is collected by a light detector 8.

The light detectors 4, 5, 8 certainly collect the transmitted light, but are arranged so as to emit an output signal corresponding to the absorbance, i.e. that part of the light radiation which is absorbed and diverged by the pulp. Between the absorbance (A) and the transmittance (T) the relation is

$$A - \log \frac{1}{T}$$

The absorbance for light radiation of a certain wavelength depends on the particle diameter. The absorbance at a constant particle concentration, e.g. measured in mg/l, has a maximum when the particle is of about equal size as the wavelength of the light. Fig. 3 shows the relation in a diagram where the sensitivity (v) of the absorbance signal has been inserted as a function of the particle diameter in logarithmic scales. Curve 1) refers to ultraviolet light with the wavelength $\lambda_{UV}$, and curve 2) refers to infrared light with the wavelength $\lambda_{IR}$. The absorbance (A) is a product of the sensitivity (v) and particle concentration (c), i.e.

$$A = v \cdot C$$

This implies that, even when the concentration of particles with a certain diameter is low, the absorbance yet can be considerable when the light ray has a wavelength of the same magnitude as the particle diameter.

In the present case, this implies that the wavelengths of the light rays transilluminating the pulp shall be as follows. One (the first) light ray shall have a wavelength of the same magnitude as the diameter of the crill particles in the pulp. In the measuring instrument practically realized by us ultraviolet light with a narrow wavelength range of about 0.254 μm is used. The second light radiation shall have a longer wavelength. Said second wavelength, however, shall be shorter than the diameter of the fibres in the pulp, preferably 0.5—5 μm. In practice, the transmission conditions in water limit the applicable interval. In the measuring instrument practically realized by us, infrared light with a narrow wavelength range of about 0.940 μm is used.

By this arrangement, the crill particles have a relatively great influence on the absorbance at the first wavelength, although the concentration of crill particles is very low relative to the concentration of fibres, measured in mg/l. At the second wavelength the influence of the crill particles on the absorbance is substantially smaller, while the influence of the fibres on the absorbance remains substantially unchanged. The difference between these two absorbance values, thus constitutes a measure of the content of crill particles in the pulp. The quotient between the two absorbance values yields a measure of the crill proportion of the pulp, i.e. the proportion of crill in the pulp. It is, of course, possible to use the absorbance values obtained for the formation of other linear combinations.

In measuring the crill proportion the result is changed if the mean diameter of the fibres is changed, since the absorbance is inversely proportional to the diameter of the fibres. This change is not desired and in order to compensate for that, the mean diameter of the fibres can be measured separately and utilized for correction of the quotient between the two absorbance values as mentioned above. This will give a crill proportion which is independent of the diameter of the fibres.

The absorbance can also be influenced by dissolved substances in the pulp suspension. In order to prevent this from interfering with the measuring of the crill content, the influence of these dissolved substances on the absorbance must be determined separately, so that the absorbance value for the pulp suspension can be corrected. This is preferably carried out by separating solid materials from the pulp sample, whereafter the absorbance measurement according to above is carried out only on the liquid. The resulting value is then subtracted from the absorbance values detected for the entire pulp sample.

In accordance with another embodiment of the method of the present invention in which the crill particles include free crill particles and crill particles partly bound to the fibres, the free crill particles are separated from the pulp suspension so to form a free crill suspension. Then the content of bound crill particles can be measured in the pulp suspension and the content of free crill particles in the free crill suspension. Alternatively first and second free crill absorbances can be measured in the free crill suspension and utilized to correct the first and second absorbance in order to determine the content of free and bound crill in the pulp suspension.

At the methods described above light radiation of two wavelengths is used for determining the crill content. This is per se sufficient, but it is also possible according to the invention to use one or several more wavelengths and to determine corresponding absorbance. It hereby can be possible, for example, to detect in greater detail the size distribution of the crill particles.

**Claims**

1. A method of measuring the content of fibrillary particles in a pulp containing also fibres of larger dimensions, the method comprising the steps of illuminating the pulp with light at a first and at a second wavelength, and separately detecting the light intensities originating from the pulp at these two wavelengths, characterized in that the first wavelength is selected so as to correspond to the mean diameter of the fibrillary particles, the second wavelength is selected so as to be longer than the first wavelength but shorter than the mean diameter of the fibres, a first and a second absorbance value are derived from the detected intensities of the light transmitted through the pulp at the first and second wavelengths, and the content of the fibrillary particles is determined from the difference between the absorbance values at the first and second wavelengths.

2. A method as defined in claim 1, characterized in that the part of the absorbance which depends on the content of solved substance in the pulp is measured separately and utilized for correcting the first and, respectively, second absorbance.

3. A method as defined in claim 1 or 2, characterized in that a quotient between the first and the second absorbance is formed for determining the proportion of fibrillary particles in the pulp.

4. A method as defined in claim 3, characterized in that the diameter of the fibres is measured separately and utilized to correct the quotient between the first and the second absorbances, so that the quotient becomes independent of the diameter of the fibres.

5. A method as defined in claim 1, wherein said fibrillary particles include free fibrillary particles and fibrillary particles partly bound to the fibres, characterized in that a suspension of the free fibrillary particles is separated from the pulp, that the content of bound fibrillary particles in the pulp is measured and the content of free fibrillary particles in the free fibrillary particle suspension is measured.

6. A method as defined in claim 5, characterized in that the content of bound fibrillary particles is determined as the difference between measured total content of fibrillary particles and measured content of free fibrillary particles.

7. A method as defined in any one of the claims 1—6, characterized in that the light at the first wavelength is ultraviolet light, and at the second wavelength infrared light.

8. A method as defined in any one of the preceding claims, characterized in that one or more further wavelengths of light are used, the or each further wavelength lying between the first and the second wavelength and in that corresponding absorbances are measured and utilized for obtaining more detailed information on the content of fibrillary particles in the pulp.

## Revendications

1. Procédé pour mesurer la teneur en particules fines en forme de fibrilles dans une pâte brute contenant également des fibres de dimensions plus importances, selon lequel on réalise un éclairement au travers de la pâte, à l'aide d'une lumière d'une première et d'une seconde longueurs d'ondes, et on détecte séparément les intensités lumineuses provenant de la pâte pour ces deux longueurs d'ondes, caractérisé en ce que la première longueur d'ondes est choisie de façon à correspondre au diamètre moyen des particules en forme de fibrilles, la seconde longueur d'ondes est choisie de manière à être plus importante que la première longueur d'ondes, mais plus courte que le diamètre moyen des fibres, en ce qu'une première et une seconde valeurs du pouvoir absorbant sont dérivées des intensités détectées de la lumière transmise au travers de la pâte pour les première et seconde longueurs d'ondes, et en ce qu'on détermine la teneur en particules en forme de fibrilles à partir de la différence entre les pouvoirs absorbantes pour les première et seconde longueurs d'ondes.

2. Procédé selon la revendication 1, caractérisé en ce que la partie du pouvoir absorbant qui dépend de la teneur en substances dissoutes dans la pâte est mesurée séparément, et elle est utilisée pour corriger le premier, et, respectivement, le second pouvoir absorbant.

3. Procédé selon l'une des revendications 1 ou 2, caractérisé en ce qu'on forme un quotient entre le premier et le second pouvoirs absorbants, afin de déterminer la proportion de particules fines dans la pâte à papier.

4. Procédé selon la revendication 3, caractérisé en ce que le diamètre des fibres est mesuré séparément, et en ce qu'il est utilisé pour corriger le quotient entre le premier et le second pouvoirs absorbants, afin que ce quotient devienne indépendant du diamètre des fibres.

5. Procédé selon la revendication 1, selon lequel les particules fines en forme de fibrilles comprennent des particules libres et des particules qui sont liées partiellement aux fibres, caractérisé en ce qu'on sépare de la pâte une suspension de particules fines libres, en ce qu'on mesure la teneur de la pâte en particules fines liées, et en ce qu'on mesure la teneur en particules fines libres dans la suspension de particules fines libres.

6. Procédé selon la revendication 5, caractérisé en ce qu'on détermine la teneur en particules fines liées, en tant que différence entre la teneur totale mesurée des particules fines et la teneur mesurée en particules fines libres.

7. Procédé selon l'une quelconque des revendications 1 à 6, caractérisé en ce que la lumière ayant la première longueur d'ondes est une lumière ultraviolette, et en ce que la lumière ayant la second longueur d'ondes est une lumière infrarouge.

8. Procédé selon l'une quelconque des revendications précédentes, caractérisé en ce qu'on utilise une ou plusieurs longueurs d'ondes supplémentaires, dont les valeurs sont comprises entre celles des première et seconde longueurs d'ondes, et en ce que les pouvoirs absorbants correspondants sont mesurés et utilisés pour obtenir des informations plus détaillées sur la teneur en particules fines de la pâte.

## Patentansprüche

1. Verfahren zum Messen des Gehaltes an Feinfaserteilchen in einer Pulpe, die auch Fasern mit grösseren Abmessungen enthält, welches Verfahren die Schritte der Beleuchtung der Pulpe mit Licht mit einer ersten und einer zweiten Wellenlänge und der separaten Erfassung der Lichtintensitäten, die von der Pulpe bei diesen beiden Wellenlängen kommen, umfasst, dadurch gekennzeichnet, dass die erste Wellenlänge so gewählt ist, dass sie dem mittleren Durchmesser der Feinfaserteilchen entspricht, die zweite Wellenlänge so gewählt ist, dass sie länger als die erste Wellenlänge, jedoch kürzer als der mittlere Durchmesser der Fasern ist, ein erster und ein zweiter Wert des Absorptionsvermögen aus den erfassten Intensitäten des durch die Pulpe hindurchgegangenen Lichtes mit der ersten und der zweiten Wellenlänge abgeleitet wird un der Gehalt an Feinfaserteilchen aus dem Unterschied zwischen den Werten des Absorptionsvermögens bei der ersten und der zweiten Wellenlänge bestimmt wird.

2. Verfahren nach Anspruch 1, dadurch gekennzeichnet, dass der Teil des Absorptionsvermögens, der vom Gehalt eines gelösten Stoffes in der Pulpe abhängt, separat gemessen und dazu benutzt wird, das erste und jeweils das zweite Absorptionsvermögen zu korrigieren.

3. Verfahren nach Anspruch 1 oder 2, dadurch gekennzeichnet, dass ein Quotient zwischen dem ersten und zweiten Absorptionsvermögen gebildet wird, um den Anteil der Feinfaserteilchen in der Pulpe zu bestimmen.

4. Verfahren nach Anspruch 3, dadurch gekennzeichnet, dass der Durchmesser der Fasern separat gemessen und dazu benutzt wird den Quotienten zwischen dem ersten und dem zweiten Absorptionsvermögen zu korrigieren, so dass der Quotient unabhängig vom Durchmesser der Fasern wird.

5. Verfahren nach Anspruch 1, wobei die Feinfaserteilchen freie Feinfaserteilchen und Feinfaserteilchen einschliessen, die teilweise an die Fasern gebunden sind, dadurch gekennzeichnet, dass eine Suspension der freien Feinfaserteilchen von der Pulpe abgetrennt wird, dass der Gehalt der gebundenen Feinfaserteilchen in der Pulpe gemessen wird und dass der Gehalt an freien Feinfaserteilchen in der Suspension der freien Feinfaserteilchen gemessen wird.

6. Verfahren nach Anspruch 5, dadurch gekennzeichnet, dass der Gehalt an gebundenen Feinfaserteilchen als Unterschied zwischen dem gemessenen Gesamtgehalt an Feinfaserteilchen und dem gemessenen Gehalt an freien Feinfaserteilchen bestimmt wird.

7. Verfahren nach einem der Ansprüche 1 bis 6, dadurch gekennzeichnet, dass das Licht mit der ersten Wellenlänge ultraviolettes Licht ist, und dass das Licht mit der zweiten Wellenlänge infrarotes Licht ist.

8. Verfahren nach einem der vorhergehenden Ansprüche, dadurch gekennzeichnet, dass Licht mit einer weiteren oder mehreren weiteren Wellenlänge benutzt wird, wobei die weitere oder jede weitere Wellenlänge zwischen der ersten und der zweiten Wellenlänge liegt, und dass die entsprechenden Absorptionsvermögen gemessen und dazu benutzt werden, eine mehr ins einzelne gehende Information über den Gehalt an Feinfaserteilchen in der Pulpe zu erhalten.

FIG.1

FIG.2

FIG.3

sensitivity
of absorbency
signal

log

1)
2)
1) 2)

λUV λIR

log

particle diameter